(19) Europäisches Patentamt
European Patent Office
Office européen des brevets

(11) EP 1 972 611 A1

(12) EUROPEAN PATENT APPLICATION
published in accordance with Art. 158(3) EPC

(43) Date of publication:
**24.09.2008 Bulletin 2008/39**

(21) Application number: **06840733.7**

(22) Date of filing: **30.12.2006**

(51) Int Cl.:
***C07C 49/543*** (2006.01) ***C07C 39/23*** (2006.01)
***C07H 1/08*** (2006.01) ***C07H 15/20*** (2006.01)
***A61K 31/12*** (2006.01) ***A61K 31/09*** (2006.01)
***A61P 35/00*** (2006.01)

(86) International application number:
**PCT/CN2006/003703**

(87) International publication number:
**WO 2007/076704 (12.07.2007 Gazette 2007/28)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.12.2005 CN 200510136656**

(71) Applicants:
- **Central South University**
**Yuelu Changsha**
**Hunan 410013 (CN)**
- **Dietetech Investment Ltd**
**Chai Wan**
**Hong Kong (CN)**

(72) Inventors:
- **ZHOU, Ying Jun**
**Changsha, Hunan 410013 (CN)**
- **SHI, Yuenian, Eric**
**Heights, New York 11577 (US)**

(74) Representative: **Grättinger & Partner (GbR)**
**Wittelsbacherstrasse 5**
**82319 Starnberg (DE)**

# (54) ARYL DIHYDRO-NAPHTHALENE COMPOUNDS, THEIR PREPARATION AND THEIR USE AS AKT INHIBITOR FOR THE PREVENTION AND TREATMENT OF CANCER

(57) The present invention discloses 9 Aryldihydronnaphthalenes that inhibit Akt activation. The invention also provides compositions composing such inhibitory compounds and methods of inhibiting Akt activity by administrating the compounds to patients with cancer.

EP 1 972 611 A1

## Description

## FIELD OF THE INVENTION

This invention discloses a novel group of aryldihydronnaphthalenes compounds with inhibitory activity towards one or more of the isoforms of the serine /threonine kinase, Akt (also known as PKB; hereinafter referred to as "Akt"). The present invention also relates to pharmaceutical compositions comprising aryldihydronnaphthalenes compounds and methods of using the said compounds in treatment of cancer.

## BACKGROUND OF THE INVENTION

Lignan is a class of compounds consisting of dimmers formed from the polymerization of derivatives of diphenylpropanoid (C6-C3). Lignans can be found in various sources, and more 200 compounds of lignan have been identified so far. Recently, trimer and tetramer are also discovered.

Since more and more lignans have been identified with novel structures, they are grouped into lignans and neolignans. When 2 units of diphenylpropanoid (C6-C3) are connected through β-carbon, the molecules are classified as lignans. When the connection is not through β-carbon, the compounds are classified as neoliganas. Recently a different classification method has also been developed by grouping phenylpropanoids with oxidized γ-carbon as lignans (consisting of two units of cinnamic acid, cinnamic aldehyde or cinnamic alcohol) and by grouping phenylpropanoids with non-oxidized γ-carbon as neolignans.

Based on the general structures of lignans, there is another classification method, which includes the following 12 subclasses: 1) dibenzylbutanes, 2) dibenzyltyrolactones, 3) arylnaphthalenes, 4) tetrahydrofurans, 5) furofurans, 6) dibenzocyclooctenes, 7) benzofurans, 8) bicyclo[3,2,1]octanes, 9) Benzol parallel dioxanes, 10) spirodienones, 11) biphenylenes, 12) sesquilignans and dilignans. Among these subclasses, arylnaphthalenes (subclass 3) has a following general structure formula (II).

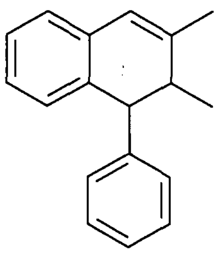

(II)

A.S.CHAVLA et al (PHYTOCHEMISTRY Vol.31. No.12. pp 4378-4379, 1992) reported the isolation of several compounds with a general structure (III) from the seeds of vitex (Vitex negundo L) in which the R is H or Ac. In this study, the author only reported the isolation and the molecule structure of the compounds.

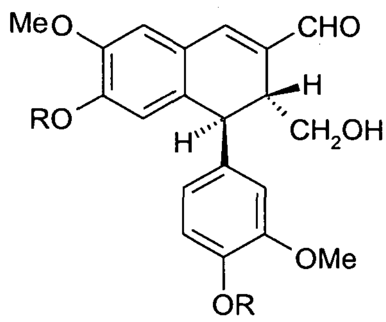

R
1 H
2 Ac

(III)

Masateru Onoet et al reported the isolation of 7 lignan compounds from the seeds of Vitex (Vitex negundo L.) (Journal of Natural Product,2004,67,2073-2075). Two of these compounds are arylhydronnaphalenes characterized by structure IV. In addition to the isolation and structure characterization, the author also reported the study results on the antioxidant activity of the compounds.

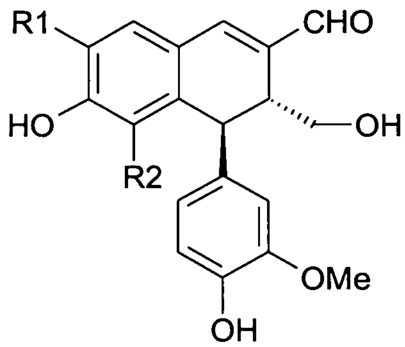

1:R1=H,R2=OCH3
2:R1=OCH3,R2=H

(IV)

It is known that one group of lignan: podophyllotoxins (aryltetralins) have anti tumor activity. For example, a patent (EP0711767) disclosed the following compound (V):

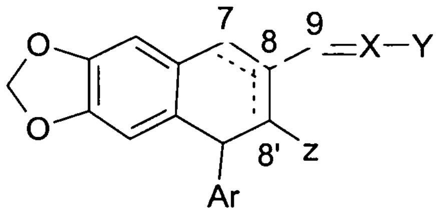

(V)

Ar = 3, 4, 5 - tri-alkoxy phenyl, or 4-hydroxy-3,5-bis-alkoxy phenyl; X = O, S, N, C ( =CH- or ≡CH-); Y = H , alkyl, alkenyls, Naphthene,phenyl, -OH, alkoxy, -NH2, -NHR(R=alkyl), -NR2(R=alkyl), cyclane substituted amino group, cyclane substituted fragrant amino group, cyclane substituted acyl group, cyclane substituted carboxy group, cyclane substituted carbalkoxy, cyclane substituted aryloxycarbonyl group. Dotted lines indicate double bound between position 7 and 8 or

8 and 8'.

Podophyllotoxins has been studied intensively in recent years. Some of the compounds have entered clinical testing stage as anti-tumor agents, such as Etoposide (VP-16) and Teniposide (VM-26). The current structure-function relationship indicates that C4 is a good position to be modified (Issell BF, et al, Etoposide [Vp-16] Current Status and New Development [M]. Academic Press, New York, 1984).

It is now well understood that a reduced capacity of tumor cells of undergoing cell death through apoptosis plays a key role in tumor pathogenesis as well as therapeutic treatment failure of cancer. Indeed, tumor cells frequently display multiple alterations in signal transduction pathways leading to either cell survival or apoptosis. In mammals, the pathway based on phosphoinositide-3-kinase (PI3K)/Akt conveys survival signals of extreme importance and its down-regulation, by means of pharmacological inhibitors of Akt, considerably lowers resistance to various types of therapy for solid tumors.

Akt is a cell survival-promoting oncoprotein. This oncoprotein has attracted much attention from researchers because of its central role in regulating cell proliferation and survival. It is abnormally activated in many human malignancies such as breast, prostate, lung, pancreatic, liver, ovarian and colorectal cancers. Increased activation is also linked with tamoxifen resistance, chemo drug and radiation resistance, and apoptosis resistance. Akt activation and over expression has been shown to be tightly linked with increased invasion and angiogenesis signaling, and to be correlated with poorer outcomes in human prostate and breast cancers. This oncoprotein appears to be an extremely important clinical target for reducing tumor drug resistance and stopping tumor invasion. Akt inhibitor can be used as an adjunctive agent in chemotherapy to reduce the drug resistance or simply as a therapeutic agent to attack cancer cells directly.

Akt is activated by phosphorylation events occurring in response to PI3K signaling. PI3K phosphorylates membrane inositol phospholipids, generating the second messengers phosphatidyl-inositol 3,4,5-trisphosphate, which have been shown to bind to the PH domain of Akt. The current model of Akt activation proposes recruitment of the Akt to the membrane by 3'-phosphorylated phosphoinositides, where phosphorylation of the regulatory sites of Akt by the upstream kinases occurs.

Inhibition of Akt activation and its activity can be achieved by blocking PI3K with inhibitors such as LY294002 and wortmannin. However, PI3K inhibition has the potential to indiscriminately affect not only Akt but also other PH domain-containing signaling molecules that are dependent on Pdtlns (3,4,5)- P3, such as the Tec family of tyrosine kinases. Furthermore, it has been disclosed that Akt can be activated by growth signals that are independent of PI3K.

Alternatively, Akt activity can be inhibited by blocking the activity of the upstream kinase PDK1. The compound UCN-01 is a reported inhibitor for PDK1. Again, inhibition of PDK1 would result in inhibition of multiple protein kinases whose activities depend on PDK1, such as atypical PKC isoforms, SGK, and S6 kinases. Small molecule inhibitors of Akt are useful in the treatment of tumors, especially those with activated Akt.

## SUMMARY OF THE INVENTION

The present invention discloses 9 compounds that inhibit Akt activation. The invention also provides compositions composing such inhibitory compounds and methods of inhibiting Akt activity by administrating the compounds to patients with cancer.

## BRIEF DESCRIPTION OF THE DRAWINGS

These and other features and advantages of this invention will be evident from the following detailed description of preferred embodiments when read in conjunction with the accompanying drawings in which:

Figure 1 demonstrates the synthesis strategy to generate various compounds by modification of VB-1. Synthesis route a: VB-1 reacts with halogenated paraffin. Synthesis route b: A mixture of VB-1, acetic anhydride and sodium acetate, is stirred over water bath overnight, and followed with extraction by AcOEt. Synthesis route c: to oxidize VB-1 with Tollens reagent. Synthesis route d: VB-1 reacts with γ-hydroxylthiobutyric acid. Synthesis route e: step 1, to form quaternary alkylphosphonium salt by refluxing of a mixture of triphenyl phosphine and a halogenated paraffin; step 2, to transforme the quaternary alkylphosphonium salt into a phosphorus ylide under n-BuLi condition; step 3, to react VB-1 with a phosphorus ylide. Synthesis route f: to dissolve VB-1 in dry EtOH, to add primary amines, to stir and heat the mixture heat until the reaction is completed. Excessive amines are removed by acid-wash, column chromatograph or preparative TLC to get pure compound.

Figure 2 demonstrates the suppressive effect of VB-2 on the implanted CoC1 tumor growth in mice (Balb/c-nu).

Figure 3 shows the inhibition of Akt activation in human breast cancer cells by Compound VB-1 and VB-2

Figure 4 presents the induction of apoptosis in human breast cancer cells by VB-1

## DETAILED DESCRIPTION OF THE INVENTION

The present invention discloses a general structure formula for arylhydronnaphthalenes:

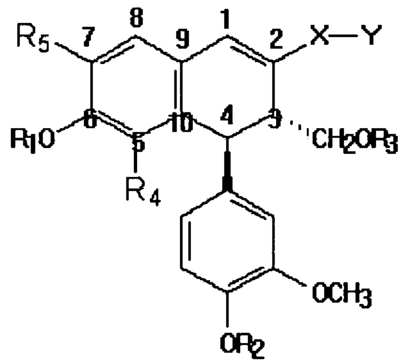

(I)

whereas x is C=O, C=S, C=N, methylene(-$CH_2$-), or alkenyl (-C=CH-; Y is hydrogen, hydroxy, amino, alkyl or nothing; $R_1$ and $R_2$ could be hydrogen, alkyl, alkenyl, glucosyl, glucuro, and aryl; $R_3$ is hydrogen or glucosyl; $R_4$ is methoxy or hydrogen; $R_5$ is methoxy or hydrogen; the configuration of 3 and 4 on core structure are R and S (3R,4S).

The compounds can be extracted with 40% ethanol from the seeds of vites (Vitex negundo L.). The concentrated extract are mixed with polyamide powder and loaded on a polyamide column. The column is eluted in a step-gradient of water/ethanol in the order of 10:90, 25:75, 40:60, 60:40 and 95:5. The 25% ethanol fraction is concentrated and loaded on a size exclusion column (resin), the column is eluted with ethanol or water saturated ethyl acetate. The concentrated eluent is loaded on to another size exclusion column and eluted with methanol. The crystallized substance can be harvested from the concentrated eluent.

The following compounds have been isolated : 6-hydroxy-4-(4-hydroxy-3-methoxyphenyl)-3-hydroxymethyl-7-methoxy-3,4-dihydro-2-naphthaldehyde (VB-1), 6-hydroxy-4-(4-hydroxy-3-methoxyphenyl)-3-hydroxymethyl-5-methoxy-3,4dihydro-2-naphthaldehyde (VB-2), and 6-hydroxy-4-(4-hydroxy-3-methoxyphenyl)-3-glucosyloxymethyl-7-methoxy-3,4-dihydro-2-naphthaldehyde (VB-3).

The aforementioned VB-1 was given to fasted rat by gavage. Every 24 hr, the rat urine and dropping were collected and the samples were mixed with methanol. The methanol extract from rat dropping was concentrated and processed further with a macro-pore absorptive resin (AB-8), the resin was eluted with ethanol and water. The 30 % ethanol fraction was further processed with gel filtration column. The rat metabolized VB-1 was collected from water elution. In the examples section, the invention discloses the structure of these metabolites: 6-hydroxy-7-methoxy-4-(4-hydroxy-3-methoxyphenyl)-2,3-dihydroxymethyl-3,4-dihydro(3R,4S)-2-hydroynaphthalene (VB-4), 6-hydroxy-3-hydroxymethyl-4-(3-methoxyphenyl)-7-methoxy-3,4-dihydro-2-naphthaldehyde-4'-O-β-D-glucopyranosiduronic acid (VB-5). Both the metabolites (VB-4) and non-metabolized (VB-1) are the members of aryldihydronnaphthalens.

Additionally, many derivatives can be derived from VB-1. Figure 1 illustrates the modification mstrategies to get various derivatives from VB-1.

The present invention discloses the following compounds: 6-hydroxy-4-(4-hydroxy-3-methoxyphenyl)-3-hydroxymethyl-7-methoxy-3,4-dihydro-2-naphthaldehyde (VB-1), 6-hydroxy-4-(4-hydroxy-3-methoxyphenyl)-3-hydroxymethyl-5-methoxy-3,4dihydro-2-naphthaldehyde (VB-2), 6-hydroxy-4-(4-hydroxy-3-methoxyphenyl)-3-glucosyloxymethyl-7-methoxy-3,4-dihydro-(3R,4S)-2-naphthaldehyde (VB-3)6-hydroxy-7-methoxy-4-(4-hydroxy-3-methoxyphenyl)-2,3-dihydroxymethyl-3,4-dihydro(3R,4S)-2-hydroynaphthalene (VB-4), 6-hydroxy-3-hydroxymethyl-4-(3-methoxyphenyl)-7-methoxy-3,4-dihydro-2-naphthaldehyde-4'-O-β-D-glucopyranosiduronic acid (VB-5), 6-methoxy-4-(4-methoxy-3-methoxyphenyl)-3-hydroxymethyl-7-methoxy-3,4dihydro-2-naphthaldehyde (VB-6), 6,7-dimethoxy-4-(4-hydroxy-3-methoxyphenyl)-3-hydroxymethyl-3,4-dihydro-2-naphthaldehyde (VB-7); 6-butyroxy-4-(4-butyroxy-3-methoxyphenyl)-3-butyroxymethyl-7-methoxyl-3,4-dihydro-2-naphthaldehyde (VB-8); 6-acetoxy-4-(4-acetoxy-3-methoxyphenyl)-3-acetoxymethyl-7-methoxyl-3,4dihydro (3R, 4S)-2-naphthaldehyde (VB-9).

The test results indicate that the disclosed compounds have inhibitory activity on various tumor cells: COC1, SMMC-7721, MCF-7, and HT-29.

Although the disclosed compounds are well established as Akt inhibitor, it should be pointed out that the present invention provided additional biological activity of the disclosed compounds. The biological activities include: inhibition of cell proliferation, anti-tumor, anti-angeonesis, inhibition of blood vessel epilia cell growth or tumor cell growth, suppression of malignant tumor, and promotion of cell aprotosis. These activities are possibly not Akt related and no efforts

have been made to understand the possible mechanisms.

The present invention presents experimental anti-tumor effects for the disclosed compounds. The invention does not address the pharmacological mechanism of the disclosed compounds. The disclosed compounds are useful to treat various tumor occurred to parenchymatous organs, including colon cancer, pancreatic cancer, breast cancer, prostate cancer, bone cancer, liver cancer, lung cancer, carcinoma of testis, skin cancer, stomach cancer, endometrial cancer, ovary cancer, brain tumor, and leukemia. Also, the disclosed compounds are useful to treat cancers with loss of PTEN or aberrant expression of Akt, which include brain cancer, breast cancer, bone cancer, endometrial cancer, liver cancer, lung cancer, prostate cancer, kidney cancer, bladder cancer, colon cancer, pancreatic cancer, leukemia, and soft tissue cancer.

Accordingly, the present invention also provides a pharmaceutical composition comprising one or more the disclosed anrylhydronnaphthalenes for treating various cancers.

**EXAMPLE 1**

**The preparation of 6-hydroxy-4-(4-hydroxy-3-methoxyphenyl)-3-hydroxymethyl-7-methoxy-3,4-dihydro-(3R, 4S)-2-naphthaldehyde (VB-1)**

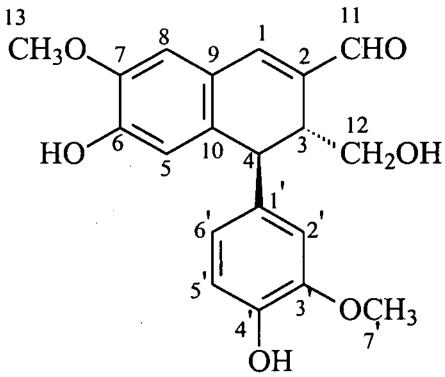

VB-1

The seeds (Vitex negundo L.) were extracted 3 times with 40% ethanol.The concentrated extract was mixed with polyamide of 30 - 60 mesh, and loaded on a polyamide column (80-100 mesh). The column was eluted with step gradient method with increased concentration of ethanol (10, 25, 40, 60 and 95 % of ethanol). After concentration, the eluate of 25% ethanol was passed through a large pore size resin column washing with 40% ethanol. The concentrated eluate was loaded on to a gel filtration column (Sephadex LH-20) and eluted with 40% methanol. The eluate from gel filtration column was concentrated for crystallization. After column, the crude crystal was recrystalized with methanol to get pure VB-1 crystal. The spectroscopic analysis of VB-1 are the follows: UV $\lambda_{max}^{MeOH}$ nm: 357, 255, 209; ESI MS(m/z): 735[$2M^+$+Na], 356$[M]^+$; and nmr in table 1.

Table 1 $^{13}C$ NMR(100MHz) $^1H$ NMR(400 MHz)of VB-1

| C | $^{13}C(\delta)$ | $^1H(\delta)$ |
|---|---|---|
| 1 | 146.5 | 7.48 (1H,s) |
| 2 | 134.8 | |
| 3 | 42.3 | 3.17(1H,d,J=4.0Hz) |
| 4 | 42.3 | 4.41 (1H,s) |
| 5 | 116.9 | 6.75 (1H,s) |
| 6 | 149.4 | |
| 7 | 146.5 | |
| 8 | 112.2 | 7.13 (1H,s) |

(continued)

| C | $^{13}C(\delta)$ | $^{1}H(\delta)$ |
|---|---|---|
| 9 | 123.2 | |
| 10 | 133.0 | |
| 11 | 191.8 | 9.49 (1H,s) |
| 12 | 60.9 | 3.44 (1H,dd,J=4.4,10.4Hz,Ha)<br>3.10(1H,t,J=10.0Hz,Hb) |
| 13 | 55.2 | 3.88(3H,s) |
| 1' | 136.1 | |
| 2' | 111.0 | 6.68 (1H,d,J=2.4Hz) |
| 3' | 146.9 | |
| 4' | 144.6 | |
| 5' | 114.3 | 6.58 (1H,d,J=8.4Hz) |
| 6' | 119.3 | 6.29 (1H,dd,J=2.0,8.8Hz) |
| 7' | 55.0 | 3.70 (3H,s) |
| Solvent: Acetone | | |

## EXAMPLE 2

**The preparation of 6-hydroxy-4-(4-hydroxy-3-methoxyphenyl)-3-hydroxymethyl-5-methoxy-3,4-dlhydro(3R, 4S)-2-naphthaldehyde (VB-2)**

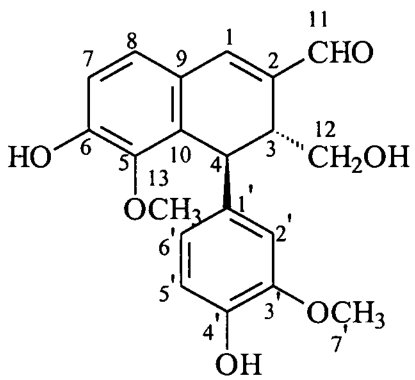

VB-2

The seeds (Vitex negundo L.) were extracted 3 times with 40% ethanol. The concentrated extract was mixed with polyamide of 30 - 60 mesh, and loaded on a polyamide column (80-100 mesh). The column was eluted with step gradient method with increased concentration of ethanol (10, 25, 40, 60 and 95 % of ethanol). Then the concentrated 25% ethanol eluate was mixed with 30 - 60 mesh of polyamide. The mixture was added to a polyamide (80-100 mesh) column and eluted with water saturated ethyl acetate. The eluate of ethyl acetate was separated on a gel filtration column (Sephadex-LH-20) using a gradient of methanol/water by increasing methanol concentration. The eluate of 40% methanol was concentrated for an overnight precipitation. The filtrated supernant was loaded on another polyamide column and eluated with chloroform/methanol (60/1). The main fraction was concentrated for over night crystallization process. The recovered crystal here was VB-2. The spectroscopic analysis of VB-2 are the follows: UV $\lambda_{max}^{MeOH}$ nm: 341, 288, 221 and nmr in table 2.

Table 2 (VB-2) $^{13}C$ NMR (100MHz) and $^{1}H$ NMR(400 MHz)

| C | $^{13}C(\delta)$ | $^{1}H(\delta)$ |
|---|---|---|
| 1 | 146.7 | 7.49 (1H,s) |
| 2 | 135.6 | |
| 3 | 43.0 | 3.21(1H,ddd,J=0.8,4.4,10.0Hz) |
| 4 | 37.4 | 4.95 (1H,s) |
| 5 | 146.7 | |
| 6 | 153.9 | 8.87 (1H,brs,6-OH) |
| 7 | 115.6 | 6.92 (1H,d, J=8.0Hz) |
| 8 | 126.7 | 7.21 (1H,d, J=8.4Hz) |
| 9 | 125.2 | |
| 10 | 133.6 | |
| 11 | 192.4 | 9.51 (1H,s) |
| 12 | 61.7 | 3.48 (1H,ddJ=4.4,10.4Hz,Ha) 3.03(1H,t,J=10.4Hz,Hb) |
| 13 | 60.3 | 3.55 (3H,s) |
| 1' | 136.0 | |
| 2' | 111.6 | 6.73 (1H,d,J=2.0Hz) |
| 3' | 147.6 | |
| 4' | 145.4 | 7.50(1H,brs,4'-OH) |
| 5' | 114.8 | 6.59 (1H,d,J=8.0Hz) |
| 6' | 120.2 | 6.34 (1H,dd,J=1.6,8.0Hz) |
| 7' | 55.6 | 3.75(3H,s) |
| Solvent: Acetone | | |

## EXAMPLE 3

**The preparation of 6-hydroxy-4-(4-hydroxy-3-methoxyphenyl)-3-glucosyloxymethyl-7-methoxy-3,4-dihydro-(3R, 4S)-2-naphthaldehyde (VB-3)**

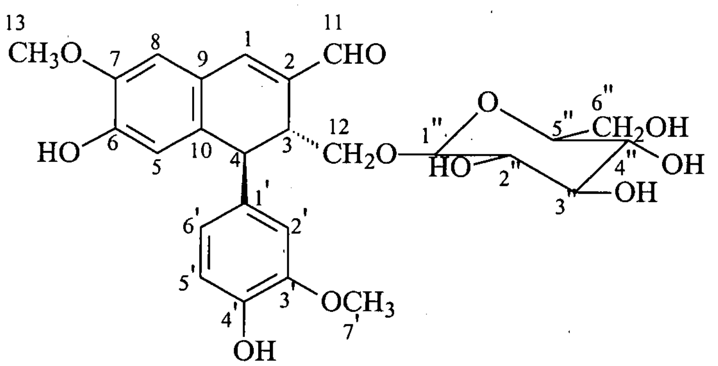

VB-3

The seeds of Vitex negundo L. were extracted 3 times with 40% ethanol. The concentrated extract was mixed with polyamide of 30 - 60 mesh, and loaded on a polyamide column (80-100 mesh). The column was eluted with step gradient method with increased concentration of ethanol (10, 25, 40, 60 and 95 % of ethanol). The eluate of 25% ethanol was concentrated and went through a large pore size resin column. The eluate of resin column was concentrated and loaded on a gel filtration column (Sephadex-LH-20). The LH-20 column was washed with a step gradient with increased methanol concentration( 10, 20, 40, 60, and 95% of methanol). The crystal of VB-3 was harvested from the concentrated eluate of 20% methanol. The spectroscopic analysis of VB-3 are the follows: (VB-3) UV $\lambda_{max}^{MeOH}$ nm:359, 256, 210; ESI MS(m/z):1036[$2M^+$], 518$[M]^+$; and nmr in table 3.

Table 3 (VB-3) $^{13}C$ NMR (100Mhz) and $^{1}H$ NMR(400 MHz)

| C | $^{13}C$ (δ) | $^{1}H$ (δ) |
|---|---|---|
| 1 | 147.1 | 7.51 (1H,s) |
| 2 | 133.3 | |
| 3 | 41.7 | 3.10(1H,m) |
| 4 | 41.7 | 4.37 (1H,s) |
| 5 | 117.4 | 6.58 (1H,s) |
| 6 | 148.2 | |
| 7 | 147.1 | |
| 8 | 113.0 | 7.05 (1H,s) |
| 9 | 121.4 | |
| 10 | 132.5 | |
| 11 | 192.4 | 9.42 (1H,s) |
| 12 | 68.6 | 3.44 (1H,dd,J=5.2,10.0Hz,Ha)<br>3.00(1H,t,J=10.0Hz,Hb) |
| 13 | 55.6 | 3.80(3H,s) |
| 1' | 135.6 | |
| 2' | 111.7 | 6.60 (1H,d,J=2.0Hz) |
| 3' | 147.1 | |
| 4' | 144.4 | |
| 5' | 114.9 | 6.53 (1H,d,J=8.0Hz) |
| 6' | 119.3 | 6.23 (1H,dd,J=2.0,8.0Hz) |
| 7' | 55.6 | 3.65(3H,s) |
| 1" | 103.9 | 4.03(1H,d,J=8.0Hz) |
| 2" | 73.3 | 3.21 (1H,m) |
| 3" | 76.1 | 3.60 (1H,m) |
| 4" | 69.8 | 3.18 (1H,m) |
| 5" | 76.5 | 3.72 (1H,m) |
| 6" | 60.8 | 3.09 (2H,m) |
| Solvent: DMSO | | |

**EXAMPLE 4**

**The preparation of 6-hydroxy-7-methoxy-4-(4-hydroxy-3-methoxyphenyl)-2,3-dihydroxymethyl-3,4-dihydro (3R, 4S)-2-hydroynaphthalene (VB-4)**

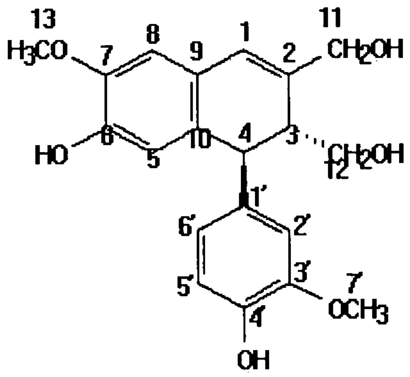

VB-4

Weighted 0.6 g of VB-1 and placed it in mortar, and added 2 ml of glycerol and grinded VB-1 gently, and added water slowly to form suspension. The final volume of the suspension was 40 ml. Each 2 ml contained 30 mg VB-1. Fed fasted rat 2 ml suspension by gavage, once a day for five days total. Every 24 hr, the rat dropping was collected. The dropping was extracted with methanol for 3 times. The concentrated extraction was absorbed by resin (AB-8). The resin was eluted with water, 10%, 20% and 30% ethanol respectively. 30% ethanol portion was processed with a gel filtration column (Sephadex LH-20) using water. About 30 mg of VB-4 was crystallized from the eluate of LH-20 column. The spectroscopic analysis of VB-4 are the follows: (VB-4) UV $\lambda_{max}^{MeOH}$ nm:358, 283, 226 and nmr in table 4.

Table 4 (VB-4) $^{13}$C NMR(100MHz) and $^{1}$H NMR(400 MHz)

| C | $^{13}$C (δ) | $^{1}$H (δ) |
|---|---|---|
| 1 | 121.2 | 6.34 (1H,s) |
| 2 | 137.0 | |
| 3 | 46.0 | 2.40(1H,dd,J=4.8,10.4Hz) |
| 4 | 42.6 | 4.02 (1H,s) |
| 5 | 117.0 | 6.52(1H,s) |
| 6 | 145.3 | 8.79 (1H,s,6-OH) |
| 7 | 145.9 | |
| 8 | 110.4 | 6.71(1H,s) |
| 9 | 124.5 | |
| 10 | 128.6 | |
| 11 | 61.2 | 3.44(1H,m),3.04(1H,m) |
| 12 | 63.6 | 4.00 (1H,dd,J=4.8,14.8Hz,Ha)<br>3.91 (1H,dd,J=4.8,14.8Hz,Hb) |
| 13 | 55.5 | 3.75(3H,s) |
| 1' | 136.7 | |
| 2' | 111.6 | 6.69 (1H,d,J=2.0Hz) |

(continued)

| C | $^{13}C$ (δ) | $^{1}H$ (δ) |
|---|---|---|
| 3' | 147.0 | |
| 4' | 144.5 | 8.60(1H,s,4'-OH) |
| 5' | 114.9 | 6.55 (1H,d,J=8.0Hz) |
| 6' | 119.5 | 6.35 (1H,dd,J=2.0,8.0Hz) |
| 7' | 55.6 | 3.66(3H,s) |
| Solvent: DMSO | | |

**EXAMPLE 5**

**The preparation of 6-hydroxy-3-hydroxymethyl-4-(3-methoxyphenyl)-7-methoxy-3,4-dihydro-2-naphthaldehyde-4'-O-β-D-glucopyranosiduronic acid (VB-5)**

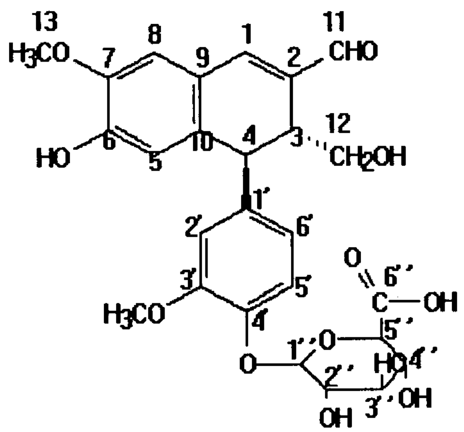

VB-5

0.6 g of VB-1 was weighted and placed in mortars. VB-1 was grinded gently with 2 ml of glycerol. Then water was added slowly to form suspension. The final volume of the suspension was 40 ml. Each 2 ml contained 30 mg VB-1. The fasted rat was fed 2 ml suspension by gavage, once a day for five days total. Urine was collected every 24 hr. The urine was mixed with methanol. After the precipitation formed, the urine/methanol solution was clarified with filtration. The concentrated urine sample was absorbed with AB-8 resin. The resin was eluted with water, 10% and 20% ethanol respectively. 10% ethanol portion was processed with a gel filtration column (Sephadex LH-20) using water. Each fraction was monitored with a thin layer chromatograph using VB-5 standard as indicator. Pool all fractions containing VB-5. The concentrated pool was further purified with reverse phase column (C-18) and the process yielded a total 19 mg of pure VB-5. The spectroscopic analysis of VB-5 are the follows: UV $\lambda_{max}^{MeOH}$ nm:357, 320, 255, 205; and nmr in table 5.

Table 5 (VB-5) $^{13}C$ NMR(100MHz) and $^{1}H$ NMR (400 MHz)

| C | $^{13}C$(δ) | $^{1}H$(δ) |
|---|---|---|
| 1 | 146.6 | 7.51 (1H,s) |
| 2 | 134.2 | |
| 3 | 41.8 | 3.03(1H,dd,J=4.0,10.4 Hz) |
| 4 | 41.8 | 4.31 (1H,s) |
| 5 | 117.3 | 6.68 (1H,s) |

(continued)

| C | $^{13}C(\delta)$ | $^{1}H(\delta)$ |
|---|---|---|
| 6 | 149.7 | |
| 7 | 146.6 | |
| 8 | 113.2 | 7.13 (1H,s) |
| 9 | 122.5 | |
| 10 | 132.3 | |
| 11 | 192.2 | 9.46 (1H,s) |
| 12 | 60.5 | 3.30 (2H,m) |
| 13 | 55.6 | 3.82(3H,s) |
| 1’ | 139.1 | |
| 2’ | 112.0 | 6.68 (1H,s) |
| 3’ | 148.8 | |
| 4’ | 144.4 | |
| 5’ | 115.5 | 6.82 (1H,d,J=8.4Hz) |
| 6’ | 118.8 | 6.21 (1H,dd,J=2.0,8.4Hz) |
| 7’ | 55.6 | 3.68(3H,s) |
| 1" | 100.0 | 4.92(1H,d,J=7.2Hz) |
| 2" | 72.8 | 3.22 (1H,m) |
| 3" | 75.9 | 3.22 (1H,m) |
| 4" | 71.2 | 3.30 (1H,m) |
| 5" | 75.3 | |
| 6" | 169.9 | 12.61 (1H,brs,6"-OH) |
| Solvent: DMSO | | |

**EXAMPLE 6**

**The preparation of 6-methoxy-4-(4-methoxy-3-methoxyphenyl)-3-hydroxymethyl-7-methoxy-3,4dihydro-2-naphthaldehyde (VB-6)**

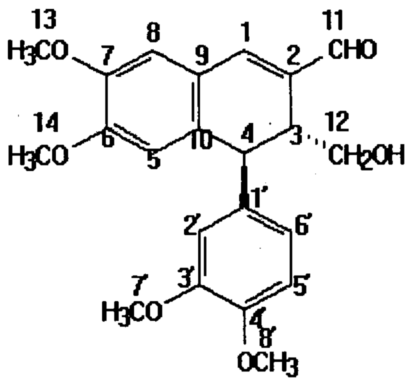

VB-6

50 mg of VB-1 was dissolved with 1.1 ml of 0.3 N NaOH. After adding dimethyl sulphate 41μl, the solution was agitated for 10 hours under ice water batch. After 10 hr reaction process, the sample was loaded on to a gel filtration column(HW-$C_{40}$). The column was washed by a step gradient of methanol/water with increased methanol concentration. A 33 mg of VB-6 was harvested from 40% methanol eluate. The spectroscopic analysis of VB-6 are the follows UV $\lambda_{max}^{MeOH}$ nm:353, 285, 252, 232, 213; and nmr in table 6.

Table 6 (VB-6) $^{13}C$ NMR(100MHz) and $^{1}H$ NMR (400 MHz)

| C | $^{13}C(\delta)$ | $^{1}H(\delta)$ |
|---|---|---|
| 1 | 146.6 | 7.49 (1H,s) |
| 2 | 136.1 | |
| 3 | 43.2 | 3.22(1H,ddd,J=0.8,4.0,10.0Hz) |
| 4 | 43.2 | 4.54 (1H,s) |
| 5 | 113.9 | 6.92(1H,s) |
| 6 | 152.4 | |
| 7 | 148.9 | |
| 8 | 112.8 | 7.15 (1H,s) |
| 9 | 124.7 | |
| 10 | 133.0 | |
| 11 | 192.3 | 9.53 (1H,s) |
| 12 | 61.8 | 3.14 (1H,m), 3.52 (1H,m) |
| 13 | 55.7 | 3.86(3H,s) |
| 14 | 55.8 | 3.83(3H,s) |
| 1' | 138.0 | |
| 2' | 112.2 | 6.70 (1H,d,J=2.0Hz) |
| 3' | 149.7 | |
| 4' | 148.4 | |
| 5' | 112.2 | 6.71 (1H,d,J=8.0Hz) |
| 6' | 119.7 | 6.39 (1H,dd,J=2.0,8.0Hz) |
| 7' | 55.6 | 3.69(3H,s) |
| 8' | 55.6 | 3.69(3H,s) |
| Solvent : Acetone | | |

## EXAMPLE 7

**The preparation of 6,7-dimethoxy-4-(4-hydroxy-3-methoxyphenyl)-3-hydroxymethyl-3,4-dihydro (3R,4S) -2-naphthaldehyde (VB-7)**

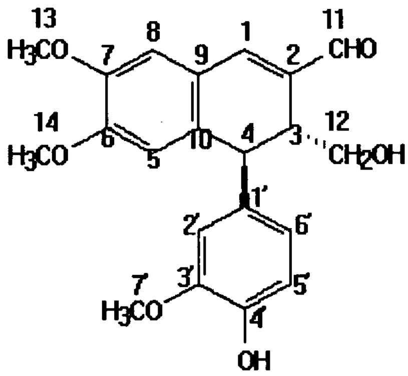

VB-7

50 mg of VB-1 was dissolved in 5 ml of acetone and followed by adding 40mg of $K_2CO_3$. While heating for reflux□26 μl of dimethyl sulphate was added. After 2 hours of reaction, acetone was evaporated. The residue was dissolved with ethyl acetate and loaded to LH-20 column. The column was eluted with a gradient of petroleum ether/ ethyl acetate (by increasing the percentage of ethyl acetate). Each fraction was monitored with a thin layer chromatograph using VB-7 standard as indicator. All fractions containing VB-7 were pooled. Total yield was 16 mg. The nmr data is in the table 7.

Table 7 VB-7 $^{13}C$ NMR(100MHz) and $^{1}H$ NMR (400 MHz)

| C | $^{13}C(\delta)$ | $^{1}H(\delta)$ |
|---|---|---|
| 1 | 146.5 | 7.48 (1H,s) |
| 2 | 136.8 | |
| 3 | 43.3 | 3.21(1H,dd,J=4.4,10.0Hz) |
| 4 | 43.3 | 4.51 (1H,s) |
| 5 | 113.9 | 6.90(1H,s) |
| 6 | 152.4 | |
| 7 | 148.9 | |
| 8 | 112.7 | 7.13 (1H,s) |
| 9 | 124.3 | |
| 10 | 133.1 | |
| 11 | 192.2 | 9.53 (1H,s) |
| 12 | 61.8 | 3.50 (2H,m) |
| 13 | 55.7 | 3.86(3H,s) |
| 14 | 55.7 | 3.83(3H,s) |
| 1’ | 136.8 | |
| 2’ | 111.5 | 6.69 (1H,d,J=2.0Hz) |
| 3’ | 147.6 | |
| 4’ | 145.4 | 7.32(1H,brs) |
| 5’ | 115.0 | 6.60 (1H,d,J=8.0Hz) |
| 6’ | 120.1 | 6.32 (1H,dd,J=2.0,8.0Hz) |
| 7’ | 55.7 | 3.72 (3H,s) |
| Solvent: Acetone | | |

**EXAMPLE 8**

**The preparation of 6-butyroxy-4-(4-butyroxy-3-methoxyphenyl)-3-butyroxymethyl-7-methoxyl-3,4-dihydro-2-naphthaldehyde (VB-8)**

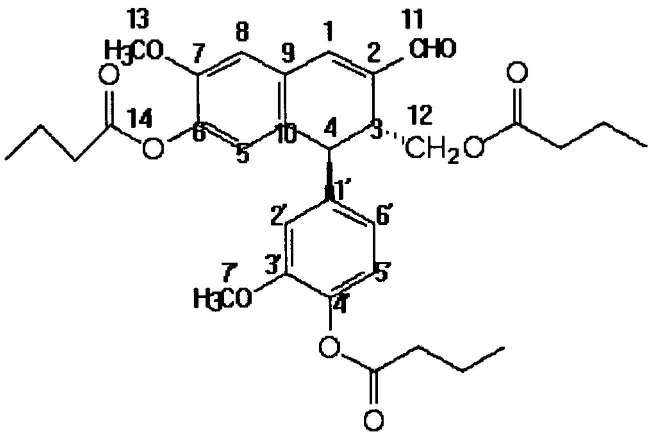

VB-8

2 ml of butyric acid was added in a round-bottom flask first, and followed by 10 ml Sulfoxide chloride. The flask was heated for reflux for 5hr and the un-reacted Sulfoxide chloride was evaporated with hot water batch. The freshly made butyryl-chloride in flask was diluted with dry tetrahydrofuran. 50 mg VB-1 was added into another flask and 1 ml butyryl- chloride in tetrahydrofuran was added drop-wise during 30 minutes period. The reaction was run for 6 hours with agitation and heat. The reaction solution was loaded on to a gel filtration column(HW-$C_{40}$)□and washed with chloroform. VB-8 was harvested from eluate.

**EXAMPLE 9**

**The preparation of 6-acetoxy-4-(4-acetoxy-3-methoxyphenyl)-3-acetoxymethyl-7-methoxyl-3,4dihydro (3R, 4S)-2-naphthaldehyde (VB-9).**

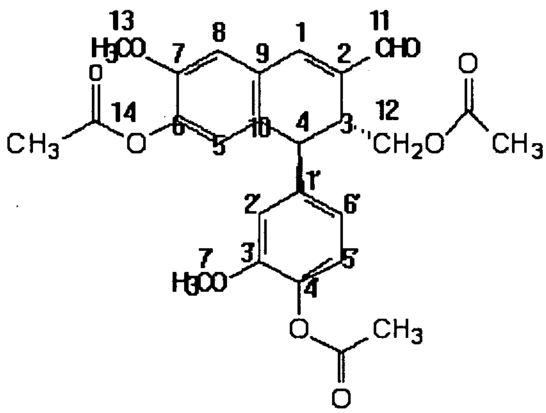

VB-9

50 mg of VB-1 was added into a 50 ml round-bottom flask. 5 ml of acetic anhydride was added into flask□and heated to 135~145°C with a condenser. The reaction in flask was ran for 1 hour. At the end of reaction, 10 ml of water was added into flask. An extraction was performed with ethyl acetate. The concentrated extract was dissolved with

chloroform and loaded it to a gel filtration column (HW-$C_{40}$). VB-9 was recovered from the eluate of chloroform.

**EXAMPLE 10**

**The inhibitory effect of VB compounds on the growth of tumor cells**

Cell lines of COC1, SMMC-7721, MCF-7and HT-29 were purchased from Chinese storage center for typical culture. Cells were cultured in RPMI1640 with 10% FCS at 37°C and 5% of CO2. Cells at log phase were harvested for the experiment.

Cells growth inhibition was measured with MTT method. Cells suspension of 1.0-1.2$\times 10^5$/ml were prepare and transferred to 96 well plate, 180μl per well. After further incubation for 2 hours, 20μl of various reagents were added to each well. The cells were back to incubator for 48 hours, and followed with addition of MTT of 20μl (5mg/ml)to each will. Additional incubation 6 hours later, the plate was centrifuged at 1000 rpm for 5 minutes. After aspirating supernatant, 100μl DMSO were added to each well and the optical density were measured at 490nm. The cells inhibition (IR) is calculated as : IR(%)=[1-A(treated)/A(control)]$\times$100% , where is A is optical density. The IR was analyzed using Calcusyn program to determine the $IC_{50}$ of each drug

The table 8, 9, 10 and 11 list some inhibition data of selected compounds on various tumor cell lines.

Table 8: Inhibition of COC1 cell growth measured by MTT method

| Compound | Concentration (μg/ml) | Inhibition (%) | $IC_{50}$(μg/ml) |
|---|---|---|---|
| VB-1 | 0.4 | 27.1 | 0.63 |
| VB-1 | 0.5 | 42.7 | |
| VB-1 | 0.6 | 48.1 | |
| VB-1 | 0.8 | 65.9 | |
| VB-1 | 1.0 | 66.9 | |
| VB-4 | 0.5 | 38.79 | |
| VB-4 | 5.0 | 40.23 | |
| VB-4 | 50.0 | 40.59 | |
| VB-5 | 0.5 | 14.30 | 371.23 |
| VB-5 | 5.0 | 28.71 | |
| VB-5 | 50.0 | 35.19 | |
| VB-2 | 0.5 | 39.87 | |
| VB-2 | 5.0 | 34.11 | |
| VB-2 | 50.0 | 39.51 | |
| VB-7 | 0.5 | 14.30 | 53.21 |
| VB-7 | 5.0 | 27.63 | |
| VB-7 | 50.0 | 49.95 | |
| VB-6 | 0.5 | 36.99 | 11.07 |
| VB-6 | 5.0 | 45.63 | |
| VB-6 | 50.0 | 57.07 | |

Table 9: Inhibition of SMMC-7721 cell growth measured by MTT method

| Sample | Conc. (μg/ml) | Inhibition (%) |
|---|---|---|
| VB-1 | 1.00 | 20.1 |
| VB-1 | 10.00 | 22.3 |

Table 10 Inhibition of MCF-7 cell growth by various compounds

| Samples | Conc.(μg/ml) | Inhibition(%) | $IC_{50}$(μg/ml) |
|---|---|---|---|
| VB-1 | 0.1 | 6.4 | 57.97 |
| VB-1 | 0.3 | 9.1 | |
| VB-1 | 1.0 | 12.7 | |
| VB-1 | 3.0 | 13.8 | |
| VB-1 | 10.0 | 41.1 | |
| VB-3 | 0.1 | 10.6 | 9262.02 |
| VB-3 | 0.3 | 11.3 | |
| VB-3 | 1.0 | 14.0 | |
| VB-3 | 3.0 | 18.5 | |
| VB-3 | 10.0 | 13.8 | |
| VB-2 | 0.1 | 10.6 | 226.22 |
| VB-2 | 0.3 | 15.8 | |
| VB-2 | 1.0 | 17.6 | |
| VB-2 | 3.0 | 26.4 | |
| VB-2 | 10.0 | 28.7 | |
| VB-5 | 0.1 | 13.6 | 471.33 |
| VB-5 | 0.3 | 22.0 | |
| VB-5 | 1.0 | 23.2 | |
| VB-5 | 3.0 | 13.6 | |
| VB-5 | 10.0 | 30.4 | |
| VB-4 | 0.1 | 19.7 | |
| VB-4 | 0.3 | 15.6 | |
| VB-4 | 1.0 | 12.3 | |
| VB-4 | 3.0 | 13.2 | |
| VB-4 | 10.0 | 24.0 | |
| VB-6 | 0.1 | 10.9 | 11.16 |
| VB-6 | 0.3 | 19.9 | |
| VB-6 | 1.0 | 22.8 | |
| VB-6 | 3.0 | 41.2 | |
| VB-6 | 10.0 | 46.4 | |

Table 11 Inhibition of HT-29 cell growth by VB-1

| Sample | Conc.(μg/ml) | Inhibition(%) |
|---|---|---|
| VB-1 | 1.00 | 22.1 |
| VB-1 | 10.00 | 32.3 |

## EXAMPLE 11

### VB-2 test results using in vivo tumor treatment model: Balb/c-nu mice inoculated with human ovarian tumor cells (CoC1)

Since VB-1 and VB-2 are two major lignan compounds isolated from Vitex lignan extract EVn-50, we investigated the tumor-suppressing effect of VB-2 on CoC1 ovary xenograft model.

Experimental Design: Human ovarian tumor cells (CoC1) were purchased from Chinese storage center for typical culture. Cells were cultured in RPMI1640 with 10% FCS. Sub-culture for every 2-3 days. Cells at log phase were harvested for the experiment. Approximately $2 \times 10^6$ CoC1 cells were injected subcutaneously into the back of the Balb/c-nu female nude mice at the age of 6-7 weeks. When tumors reached to ≥ 1000 $mm^3$ (about 30 days), mice bearing similar size tumors were divided into 2 groups: control (NS) and VB-2 treated group. Each group has 4 mice.

VB-2 was dissolved into DMSO and further diluted with normal saline. Mice were injected (interperitoneal i.p) either with saline or VB-2 at the dose of 10 mg/kg. Treatment groups received drugs every other day until the termination of the experiment. All mice were sacrificed at day 20 after the first treatment. Tumor size was determined by three-dimensional measurements (mm) using a caliper. Only measurable tumors were used to calculate the mean tumor volume for each clone at each time point. Each point represents the mean of tumors ± SE. Statistical comparisons for tumor size in treated mice relative to control mice indicate: ** $p < 0.01$.

Results: Before treatment (Table 12), there was no difference in term tumor volume between control and VB-2 groups, and these mice were ready for the treatment evaluation. After 5 days treatment (Table 13), the tumors under VB-2 treatment were slightly smaller the control. Table 14 demonstrates that after 10 days treatment, the tumors under VB-2 treatment were much smaller than the tumor of control group. The suppression rate was 66.5% (p<0.05). As shown by Table 15 that after 15 days treatment, the tumors under VB-2 treatment were much smaller than the tumor of control group. The suppression rate was 64.5% (p<0.05). Also as demonstrated in Table 16, after 20 days treatment, the tumors under VB-2 treatment were much smaller than the tumor of control group. The suppression rate was 55.7% (p<0.01). The table 12 to 16 are summarized in figure 2.

Table 12 Initial tumor volume ($\bar{x} \pm s$, n=4)

| Group # | Drug | Dosage (mg/kg) | Volume ($mm^3$) | Suppression rate of tumor growth (%) |
|---|---|---|---|---|
| 1 | NS | --- | 1585±1285 | --- |
| 2 | VB-2 | 5 | 1543±1466 | --- |
| F=0.12,P=1.0 | | | | |

Table 13 Tumor volume at day 5 ($\bar{x} \pm s$, n=4)

| Group # | Drug | Dosage (mg/kg) | Volume ($mm^3$) | Suppression rate of tumor growth (%) |
|---|---|---|---|---|
| 1 | NS | --- | 2756±1672 | --- |
| 2 | VB-2 | 5 | 2542±1718 | 7.8 |
| F=0.062, P=0.997 | | | | |

Table 14 Tumor volume at day 10 ($\bar{x} \pm s$, n=4)

| Group # | Drug | Dosage (mg/kg) | Volume ($mm^3$) | Suppression rate of tumor growth (%) |
|---|---|---|---|---|
| 1 | NS | --- | 6360±2691 | --- |
| 2 | VB-2 | 5 | 2131±1207* | 66.5 |
| F=2.035,P=0.122 *p<0.05 vs. Control (NS) | | | | |

Table 15, Tumor volume at day 15 ($\bar{x}\pm s$, n=4)

| Group # | Drug | Dosage (mg/kg) | Volume ($mm^3$) | Suppression rate of tumor growth (%) |
|---|---|---|---|---|
| 1 | NS | --- | 10156±5163 | --- |
| 2 | VB-2 | 5 | 3600±1755* | 64.5 |
| F=2.889, P=0.044 *p<0.05 vs. Control (NS). | | | | |

Table 16, Tumor volume at day 20 ($\bar{x}\pm s$, n=4)

| Group # | Drug | Dosage (mg/kg) | Volume ($mm^3$) | Suppression rate of tumor growth (%) |
|---|---|---|---|---|
| 1 | NS | --- | 13874±5652 | --- |
| 2 | VB-2 | 5 | 4495±2087** | 55.7 |
| F=3.725, P=0.017 *p<0.05, **p<0.01 vs. Control (NS) | | | | |

**Example 12**

**Inhibition of Akt activation in human breast cancer cells by Compound VB-1 and VB-2**

Experimental Design: MDA-MB-435 human breast cancer cells were cultured in the DMEM medium with 5% FCS. When cells reached approximately 50-60% confluence, cells were treated with VB-1 and VB-2 for 12 hours at the following doses: 0, 0.5 μM, 4 μM, and 8 μM. Control and treated cells are harvested and total protein was isolated, normalized, and 45 μg of total protein was subjected to Western analysis. The expression of phosphorylated Akt was determined by using a specific anti-phosphorylated Akt antibody and normalized for total Akt protein expression.

Results: As shown by Fig. 3, both VB-1 and VB-2 exert a dose-dependent inhibition of Akt activation by suppressing its phosphorylation. While both compounds have no effects on levels of total non-phosphorylated Akt protein, indicating that these compounds don't affect the Akt protein stability, compound A and B, at the dose of 4 μM, significantly block Akt phosphorylation; at the dose of 8 μM, VB-1 completely blocks Akt activation.

**Example 13**

**Induction of apoptosis in MDA-MB-435 human breast cancer cells by VB-1**

Experimental design: MDA-MB-435 human breast cancer cells were cultured in the DMEM medium with 5% FCS. Cells were treated with VB-1 at 2 μM, 4 μM and 10 μM for 3 days. Cells were collected from control and treated groups and measured with MTT method.

Results: as shown by Fig 4, VB-1 has dose dependent cell inhibition. At 10 μM, there was approximate 50 % inhibition. The growth in control group is 100%. The growth in treated groups is expressed as percentage of the control group. Each column is the mean ± SD. At 4 and 8 μM levels, p value is less than 0.005, cells growth is significantly less than the control group.

## Claims

1. A compound of aryldihydronnaphthalenes having a general structure formula (I) of:

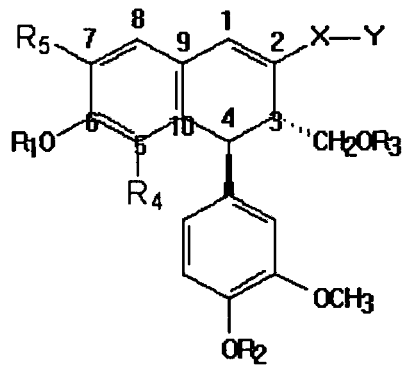

(I)

wherein, X can be C=O, C=S, C=N, methylene , or alkenyl group ; Y represents hydrogen, hydroxy, amido, alkyl group or nothing; $R_1$ and $R_2$: can be hydrogen, alkyl, alkenyl, glucosyl, glucuro, acyl group respectively; $R_3$ can be hydrogen , glucosyl or aryl group; R4 is either hydrogen or methoxy group; R5 is hydrogen or methoxy group; the hydrogen at position 3 and 4 are R and S configuration (3R and 4S) respectively.

2. A compound of claim 1, wherein the aryldihydronnaphthalenes selected from the group consisting of: 6-hydroxy-4-(4-hydroxy-3-methoxyphenyl)-3-hydroxymethyl-7-methoxy-3,4-dihydro-2-naphthaldehyde, 6-hydroxy-4-(4-hydroxy-3-methoxyphenyl)-3-glucosyloxymethyl-7-methoxy-3,4-dihydro-(3R,4S)-2-naphthaldehyde, 6-hydroxy-4-(4-hydroxy-3-methoxyphenyl)-3-hydroxymethyl-5-methoxy-3,4dihydro-2-naphthaldehyde, 6-hydroxy-7-methoxy-4-(4-hydroxy-3-methoxyphenyl)-2,3-dihydroxymethyl-3,4-dihydro(3R, 4S)-2-hydroynaphthalene, 6-hydroxy-4-(4-hydroxy-3-methoxyphenyl)-3-hydroxymethyl-7-methoxy-3,4-dihydro-2-naphthaldehyde , 6-methoxy-4-(4-hydroxy-3-methoxyphenyl)-3-hydroxymethyl-7-methoxy-3,4dihydro-2-naphthaldehyde, 6-methoxy-4-(4-methoxy-3-methoxyphenyl)-3-hydroxymethyl-7-methoxy-3,4dihydro-2-naphthaldehyde, 6-butyroxy-4-(4-butyroxy-3-methoxyphenyl)-3-butyroxymethyl-7-methoxyl-3,4-dihydro-2-naphthaldehyde, 6-acetate-4-(4-acetate-3-methoxyphenyl)-3-acetate-7-methoxy-3,4dihydro (3R, 4S)-2-naphthaldehyde.

3. A compound of claims 1 and 2, wherein the compounds are isolated directly from the seeds of Vitex negundo L., or isolated from the animal metabolized plant extract.

4. A composition comprising one or more aryldihydronnaphthalenes for treating cancers, said aryldihydronnaphthalenes having a formula (I) and thereof salt of:

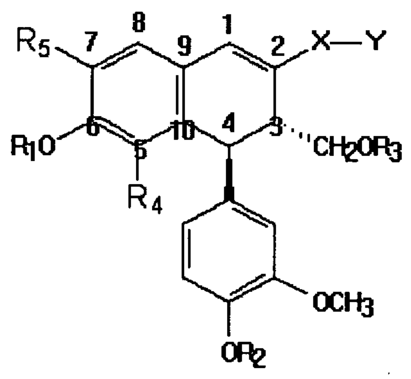

(I)

wherein, X can be C=O, C=S, C=N, methylene , or alkenyl group ; Y represents hydrogen, hydroxy, amido, alkyl group or nothing; $R_1$ and $R_2$: can be hydrogen, alkyl, alkenyl, glucosyl, glucuro, acyl group respectively; $R_3$ can be hydrogen , glucosyl or aryl group; R4 is either hydrogen or methoxy group; R5 is hydrogen or methoxy group; the hydrogen at position 3 and 4 are R and S configuration (3R and 4S) respectively; alkyls include one to three carbons, methyl, ethyl, propyl; alkenyls include two to four carbons, ethylene, propenyl, butenyl; acyls include two to four

carbons, acetyl, propionyl, butyryl.

**5.** The composition of claim 4, wherein the aryldihydronnaphthalenes is selected from the group consisting of : 6-hydroxy-4-(4-hydroxy-3-methoxyphenyl)-3-hydroxymethyl-7-methoxy-3,4-dihydro-2-naphthaldehyde, 6-hydroxy-4-(4-hydroxy-3-methoxyphenyl)-3-hydroxymethyl-5-methoxy-3,4dihydro-2-naphthaldehyde, 6-hydroxy-4-(4-hydroxy-3-methoxyphenyl)-3-glucosyloxymethyl-7-methoxy-3,4-dihydro-(3R,4S)-2-naphthaldehyde, 6-hydroxy-7-methoxy-4-(4-hydroxy-3-methoxyphenyl)-2,3-dihydroxymethyl-3,4-dihydro(3R,4S)-2-hydroynaphthalene, 6-hydroxy-3-hydroxymethyl-4-(3-methoxyphenyl)-7-methoxy-3,4-dihydro-2-naphthaldehyde-4'-O-β-D-glucopyranosiduronic acid, 6-methoxy-4-(4-methoxy-3-methoxyphenyl)-3-hydroxymethyl-7-methoxy-3,4dihydro-2-naphthaldehyde, 6,7-dimethoxy-4-(4-hydroxy-3-methoxyphenyl)-3-hydroxymethyl-3,4-dihydro-2-naphthaldehyde, 6-butyroxy-4-(4-butyroxy-3-methoxyphenyl)-3-butyroxymethyl-7-methoxyl-3,4-dihydro-2-naphthaldehyde, 6-acetoxy-4-(4-acetoxy-3-methoxyphenyl)-3-acetoxymethyl-7-methoxyl-3,4dihydro (3R, 4S)-2-naphthaldehyde and the extract mixture with 6-hydroxy-4-(4-hydroxy-3-methoxyphenyl)-3-hydroxymethyl-7-methoxy-3,4-dihydro-2-naphthaldehyde and 6-hydroxy-4-(4-hydroxy-3-methoxyphenyl)-3-hydroxymethyl-5-methoxy-3,4dihydro-2-naphthaldehyde as main components.

**6.** A composition of claim 4, wherein said cancer is melanoma, hepatocellular cancer, renal cell carcinoma, non small lung cancer, ovarian cancer, prostate cancer, colorectal cancer, breast cancer, and pancreatic cancer.

**7.** A compound of claim 1 and 2, wherein said compounds or a pharmaceutically acceptable salt thereof and the composition comprising said compounds or a pharmaceutically acceptable salt thereof with a pharmaceutical acceptable carrier are Akt inhibitors.

**8.** A compound of claim 1 and 2, wherein said compounds or a pharmaceutically acceptable salt thereof and the composition comprising said compounds or a pharmaceutically acceptable salt thereof with a pharmaceutical acceptable carrier are used for treating and preventing cancers.

**9.** A compound of claim 1 and 2, wherein said compounds or a pharmaceutically acceptable salt thereof and the composition comprising said compounds or a pharmaceutically acceptable salt thereof with a pharmaceutical acceptable carrier are Akt inhibitors or used for treating and preventing cancers.

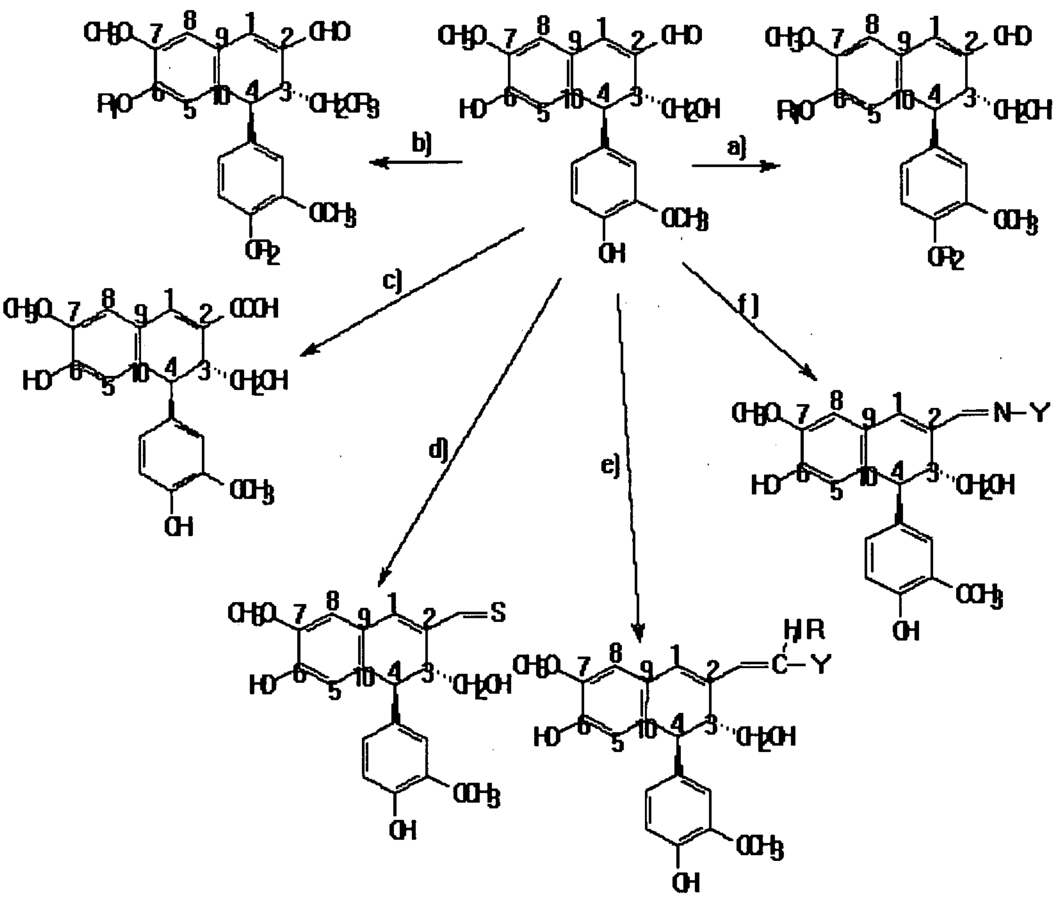


Figure 1 Modification Methods:

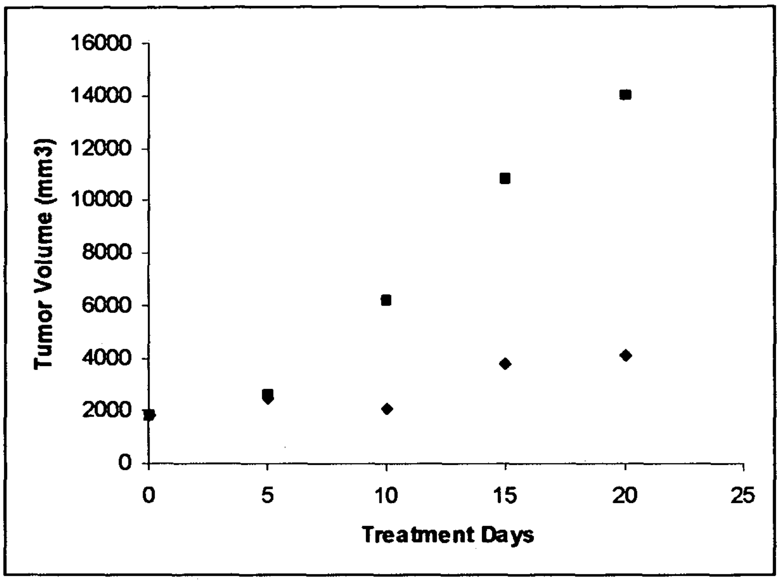


Figure 2 Tumor growth curves: —◆—: VB-2, —■— normal saline. X axle: treatment days. Y axle: tumor volume ($mm^3$).

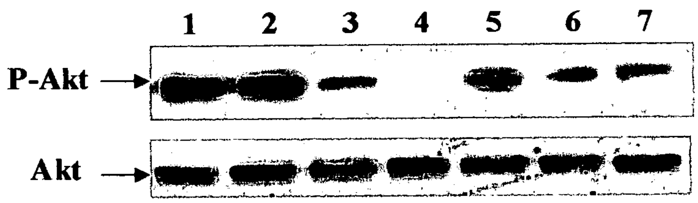


**Figure 3** **Lane 1,** Control; **lane 2,** VB-1, 0.5 μM; **lane 3,** VB-1, 4 μM; **lane 4,** VB-1, 8 μM; **lane 5,** VB-2, 0.5 μM; **lane 6,** VB-2, 4 μM; **lane 7,** VB-2, 8 μM.

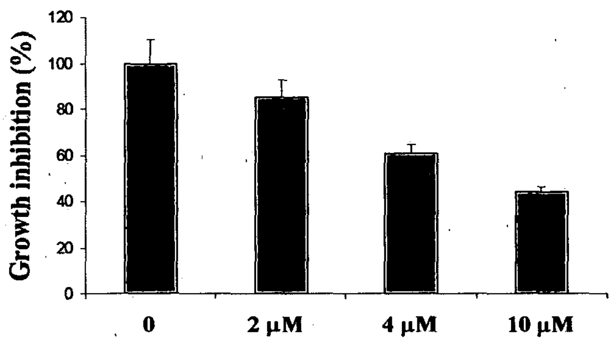


Figure 4 Induction of apoptosis in human breast cancer cells by VB-1 at 0, 2, 4 and 10 μM.

# INTERNATIONAL SEARCH REPORT

International application No.
PCT/CN2006/003703

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)

IPC: C07C, C07H A61K, A61P

Documentation searched other than minimum documentation to the extent that such documents are included in the fields searched

Electronic data base consulted during the international search (name of data base and, where practicable, search terms used)

WPI, PAJ, EPODOC, CNKI, CPRS, CAPLUS, REG

C. DOCUMENTS CONSIDERED TO BE RELEVANT

| Category* | Citation of document, with indication, where appropriate, of the relevant passages | Relevant to claim No. |
|---|---|---|
| X | A. S. CHAWLA ET-AL, A lignan from Vitex negundo seeds, Phytochemistry, 1992, 31(12), pages 4378-4379, compound 1 | 1-3 |
| X | Masateru Ono ET-AL, Lignan derivatives and a norditerpene from the seeds of vitex negundo, J. Nat. Prod., 2004, 67, pages 2073-2075, compounds 1 and 4 | 1-3 |
| X | AZHAR-UL-HAQ ET-AL, Enzymes inhibiting lignans from Vitex negundo, Chem. Pharm. Bull., 2004, 52(11), pages 1269-1272, compound 2 | 1-3 |
| X | US 6284789 B1(The Research Foundation of State University of New York) 04.Sep. 2001 (04.09.2001) compounds 98, 99, 110 and 31 in Table 2 | 1-2 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 26.Mar. 2007 (26.03.2006) | 12 · APR 2007 (12 · 04 · 2007) |
| Name and mailing address of the ISA/CN<br>The State Intellectual Property Office, the P.R.China<br>6 Xitucheng Rd., Jimen Bridge, Haidian District, Beijing, China 100088<br>Facsimile No. 86-10-62019451 | Authorized officer<br>CHEN Jun-xia 陈俊霞<br>Telephone No. 86-10-62085570 |

Form PCT/ISA /210 (second sheet) (April 2005)

# INTERNATIONAL SEARCH REPORT

| International application No. |
|---|
| PCT/CN2006/003703 |

CLASSIFICATION OF SUBJECT MATTER

C07C49/543 (2006.01) i
C07C39/23 (2006.01) i
C07H1/08 (2006.01) i
C07H 15/20 (2006.01) i
A61K31/12(2006.01) i
A61K31/09 (2006.01) i
A61P35/00 (2006.01) i

Form PCT/ISA /210 (extra sheet) (April 2005)

## INTERNATIONAL SEARCH REPORT

International application No. PCT/CN2006/003703

C (Continuation). DOCUMENTS CONSIDERED TO BE RELEVANT

| Category* | Citation of document, with indication, where appropriate, of the relevant passages | Relevant to claim No. |
|---|---|---|
| A | Yvon, Brigitte L. ET-AL，Synthesis of magnoshinin and cyclogalgravin: modified Stobbe condensation reaction，Synthesis, 2001, (10), pages: 1556-1560，compounds 8b and 9b | 1-6 |
| A | Miyazawa, Mitsuo ET-AL，(-)-Magnofargesin and (+)-magnoliadiol, two lignans from Magnolia fargesii, Phytochemistry, 1996, 42(2), pages: 531-533, compound 2 | 1-6 |

Form PCT/ISA /210 (continuation of second sheet ) (April 2005)

## INTERNATIONAL SEARCH REPORT

International application No.
PCT/CN2006/003703

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 7-9
because they relate to subject matter not required to be searched by this Authority, namely:

the subject-matter of claims 7-9 relates to the methods for treatment of the human or animal body by therapy methods.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fees, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA /210 (continuation of first sheet (2)) (April 2005)

**INTERNATIONAL SEARCH REPORT**
Information on patent family members

International application No.
PCT/CN2006/003703

| Patent Documents referred in the Report | Publication Date | Patent Family | Publication Date |
|---|---|---|---|
| US 6284789 B1 | 2001-09-04 | None | |

Form PCT/ISA /210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0711767 A


### Non-patent literature cited in the description


- **A.S.CHAVLA et al.** *PHYTOCHEMISTRY,* 1992, vol. 31 (12), 4378-4379
- *Journal of Natural Product,* 2004, vol. 67, 2073-2075
- **ISSELL BF et al.** Etoposide [Vp-16] Current Status and New Development [M. Academic Press, 1984